# EUROPEAN PATENT SPECIFICATION

(11) **EP 4 380 381 B1**
(45) Date of publication and mention of the grant of the patent: **08.07.2026**
(21) Application number: 22760997.1
(22) Date of filing: 08.08.2022
(51) Int. Cl.: A61K 38/01, A23L 33/185, A23L 33/175, A23L 33/00, A61P 21/06, A61K 31/198, A61K 38/00, A61K 31/59

(54) **NUTRITIONAL COMPOSITION FOR USE IN IMPROVING MUSCLE MASS, STRENGTH AND FUNCTION**
ERNÄHRUNGSZUSAMMENSETZUNG ZUR VEWENDUNG IN DER VERBESSERUNG DER MUSKELMASSE, MUSKELKRAFT UND MUSKELFUNKTION
COMPOSITION NUTRITIONNELLE POUR UTILISATION DANS L'AMÉLIORATION DE LA MASSE, DE LA FORCE ET DE LA FONCTION MUSCULAIRE

(30) Priority: 06.08.2021 EP 21190210
(43) Date of publication of application: 12.06.2024
(73) Proprietor: N.V. Nutricia, 2712 HM Zoetermeer (NL)
(72) Inventor: DIJK, Francina Jeannette, 3584 CT Utrecht (NL); VAN DIJK, Miriam, 3584 CT Utrecht (NL); FURBER, Matthew James Walter, 3584 CT Utrecht (NL); VAN HELVOORT, Adrianus Lambertus Bertholdus, 3584 CT Utrecht (NL)
(74) Representative: Nederlandsch Octrooibureau
(86) International application number: PCT/EP2022/072218
(87) International publication number: WO 2023/012371

(56) References cited:
- WO-A1-2013/148685
- WO-A1-2020/247753
- US-A1- 2013 210 780
- US-A1- 2019 111 083
- BRENNAN JESSICA L. ET AL: "Differential Responses of Blood Essential Amino Acid Levels Following Ingestion of High-Quality Plant-Based Protein Blends Compared to Whey Protein-A Double-Blind Randomized, Cross-Over, Clinical Trial", NUTRIENTS, vol. 11, no. 12, 6 December 2019 (2019-12-06), CH, pages 2987, XP093354842, ISSN: 2072-6643, DOI: 10.3390/nu11122987
- MATTHEW STARK ET AL: "Protein timing and its effects on muscular hypertrophy and strength in individuals engaged in weight-training", JOURNAL OF THE INTERNATIONAL SOCIETY OF SPORTS NUTRITION, BIOMED CENTRAL LTD, LO, vol. 9, no. 1, 14 December 2012 (2012-12-14), pages 54, XP021129246, ISSN: 1550-2783, DOI: 10.1186/1550-2783-9-54
- STEPHAN VAN VLIET ET AL: "The Skeletal Muscle Anabolic Response to Plant- versus Animal-Based Protein Consumption", THE JOURNAL OF NUTRITION, vol. 145, no. 9, 29 July 2015 (2015-07-29), US, pages 1981 - 1991, XP055618196, ISSN: 0022-3166, DOI: 10.3945/jn.114.204305
- BERRAZAGA INSAF ET AL: "The Role of the Anabolic Properties of Plant- versus Animal-Based Protein Sources in Supporting Muscle Mass Maintenance: A Critical Review", NUTRIENTS, vol. 11, no. 8, 7 August 2019 (2019-08-07), pages 1825, XP055875466, DOI: 10.3390/nu11081825

## Description

### Field of the invention

The invention relates to a nutritional composition comprising a specifically designed protein fraction for a human subject suffering from or at increased risk of whole body protein metabolism decline or whole body protein function decline, or for therapeutic use in improving whole body protein metabolism or whole body protein function. The invention is particularly related to prevention and/or treatment of muscle decline, particularly for therapeutic use in improving muscle mass, strength and function.

### Background of the invention

Muscle mass loss is critical to the ageing population but also for other muscle-wasting populations like oncology or hospitalized patients. The rates of protein synthesis and degradation in skeletal muscle constantly adapt in order to maintain muscle mass. Muscle protein synthesis (MPS) and muscle protein breakdown rates are highly influenced by physical activity and food intake. In healthy adults, dietary intake is generally associated with an increase in plasma concentrations of nutrients and hormones, causing an increase in protein synthesis and a decrease in protein breakdown rates. However, with ageing, humans develop an 'anabolic resistance' to the stimulation of muscle protein synthesis following meal intake. Ultimately, this could lead to net protein loss at the skeletal muscle level. A generalized reduction in skeletal muscle mass and function is called 'sarcopenia'. Frailty and sarcopenia are challenges that come with age, and are accelerated by acute or chronic disease.

Berrazaga et al. "The role of the anabolic properties of plant- versus animal-based protein sources in supporting muscle mass maintenance: a critical review" Nutrients (2019), 11, 1825 highlights the importance of optimizing protein intake while ageing, both by increasing protein quantity and improving protein quality, to overcome the reduced muscle anabolic response to food intake. It acknowledges that several studies have evaluated the effect of consuming plant-based proteins on muscle protein metabolism in young, adult and old rats, pigs, and humans, compared to animal proteins, i.e., meat, milk, and its constitutive proteins (casein and whey proteins). The majority of these studies have reported that good-quality animal proteins have a greater ability to enhance MPS rate and support muscle mass than plant-based proteins. This is also apparent from all kinds of parameters assessing food protein quality in terms of digestibility, net protein utilization and biological value, such as there is the PDCAAS and DIAAS.

Reference is made to WO2013/148685, US2019/111083, WO2020/247753 and van Vliet et al. "The Skeletal 1-15 Muscle Anabolic Response to Plant- versus Animal-Based Protein Consumption", J. Nutrition, vol. 145, no. 9, 29 July 2015 (2015-07-29) , pages 1981-1991. Both WO2013/148685 and US2019/111083 teach combinations of proteins including milk proteins. WO2013/148685 is concerned with controlling viscosity of a composition with intact pea protein to obtain improved processability, and mouthfeel to obtain improved acceptability by consumers.

When aiming to optimize protein intake while ageing, and enhancing or maintaining MPS, whey proteins are still the standard. For example, in older adults, the MPS rate was 30-40% lower following the consumption of a given quantity of soy or wheat protein hydrolysates than with whey protein isolate or micellar casein. Reference is made to Yang et al. "Myofibrillar protein synthesis following ingestion of soy protein isolate at rest and after resistance exercise in elderly men" Nutr. Metab. 2012, 9, 57. Yang et al. showed that the consumption of soy protein isolate in elderly men in resting conditions did not stimulate the MPS rate, which remained lower than that induced by the consumption of the same amount of whey protein isolate. It is remarked that leucine content in whey protein is about 12%, and only 8% in soy protein. The branched chain amino acid leucine has been shown to be a key activator of MPS through its ability to regulate mRNA translation initiation through the mTOR signalling pathway. Still, an increased administration of soy protein (and inherently the administered amount of leucine) intake did not show any effect in terms of leucine bioavailability; the above observation was reported regardless of the quantity of protein ingested (20 g or 40 g), at least not in rest. These differences in MPS between whey and soy protein might be related to a lower postprandial leucinemia and higher amino acid oxidation following the consumption of soy proteins compared to whey proteins.

However, worldwide, plant-based proteins contribute more to protein intake than animal-based proteins. Furthermore, older people generally eat less animal products, due to a blunted appetite for protein-rich foods, reduced chewing efficiency, metabolic abnormalities requiring a reduction of animal food products, and socio-economic factors. It is, therefore, necessary to develop plant-protein based foods adapted to older people's needs. Besides, plant-based diets are not just valuable for physical human health but also more environmentally sustainable than animal-based diets.

Summarizing, plant-based protein sources are rich in fiber and micronutrients, and may be valuable, but they have lower anabolic potential than animal-based proteins. Strategies to improve these properties by increasing protein intake or preferentially improving protein quality (i.e., their amino acid composition) may include selective breeding, fortifying plant-based proteins with specific essential amino acids, mixing several plant proteins, or mixing plant- with animal-based protein sources. While these strategies have been studied in younger individuals, these have yet to be examined in pathophysiological settings requiring high-quality food proteins to mitigate muscle loss.

### Summary of the invention

The scope of the claimed invention is set out in the appended claims. It is the aim of this invention to provide a nutritional composition for use in prevention and/or treatment of whole body protein metabolism decline or whole body protein function decline, or for use in improving whole body protein metabolism or whole body protein in a human subject, wherein the human subject is an-ageing human elderly subject of at least 40 years of age, who is suffering of or at high or increased risk of whole body protein metabolism decline or whole body protein function decline, and/or at (increased) risk of developing sarcopenia or frailty, and which composition is based on a sustainable, plant-derived protein blend which provides a good alternative to animal-derived compositions which are conventional in the prior art, and which typically involve casein, caseinate and/or whey protein. It serves the market's needs for sustainable alternatives to animal-based nutritional products for mitigating muscle mass loss, minimizing our foot print, without compromising on the health effects achieved with casein, caseinate and particularly whey protein.

The inventors found that such a plant-derived composition free from animal-derived protein yielding adequate numbers on muscle protein synthesis (MPS) could be obtained by combining soy and pea protein in similar amounts, provided that leucine is added to compensate for the loss of leucine bioavailability - compared to the whey protein standard - which is necessary to cover for the negative effects of anti-nutritional factors (ANFs) which are abundantly present in pea but particularly in soy protein sources. The inventors realized that these ANFs and particularly trypsin inhibitors could disadvantageously hinder any positive effects for pea and particularly soy proteins, and this loss should be compensated using free leucine.

In spite of suggestions in the art, combining different plant proteins does not yield satisfactory MPS results; it was found that MPS levels for combinations of soy and pea protein were 20% less than observed for whey protein. However, it was found that replacing some of the soy and pea protein with free leucine boosted the MPS levels of the blend comparable to those observed for whey protein, while keeping administered proteinaceous matter levels the same. The administration of increased protein concentrations or amounts is thus avoided. The results are discussed in the experimental parts. While the addition of free leucine is known to have limited effects particularly in healthy ageing, the inventors found that it was necessary to provide free leucine to a blend of soy and pea protein in order to keep the bioavailability of leucine on par with that observed for whey protein, thus overcoming any negative effects for ANF and particularly trypsin inhibitors in plant-based proteins, especially considering the trypsin inhibition activity for soy protein. Advantageously, with the soy-pea blend enriched with free leucine, there is no need to increase the protein intake compared to protein intake when using a protein fraction based on whey protein, yet it is possible to arrive at similar MPS levels.

Hence, while plant-based protein sources are typically not as anabolic as gold standard whey protein, the inventors created an engine that is equivalent to whey protein. This makes the composition a useful alternative to those in need thereof, and who prefer non-animal protein sources.

The invention particularly pertains to the non-therapeutic use of the claimed nutritional composition in prevention and/or treatment of muscle decline, particularly for use in improving muscle mass, strength and function, particularly during healthy ageing.

### List of figures

Figure 1 shows MPS in aged mice. *Significantly different compared to fasted state (P<0.05). No significant differences were found between the different blends.
Figure 2A plots the leucine bioavailability in time for the groups studied in example 1. Fasted mice did not show any increased levels of leucine; however, supplementation showed significant increase of leucine in time, where the results with addition of free leucine were most profound.

In Figure 2B the area under the curve of Figure 2A has been plotted, demonstrating that the protein supplements yielded similar leucine plasma levels for whey and the pea+soy protein blend which was enriched with free leucine.

### Detailed description of the invention

The invention targets a population of ageing human subjects of at least 40 years of age, preferably human subjects of at least 45 years of age, more preferably human subjects of at least 50 years of age, most preferably at least 55 years of age. The human subjects are preferably healthy, and preferably at (increased) risk of developing sarcopenia and/or frailty. In one embodiment, the human subject is a person not suffering from COPD.

The subject is an elderly human. In this respect, it is submitted that in the context of this application, an elderly human is a person of the age of 50 years or more, in particular of the age of 55 or more, more in particular of the age of 60 or more, more in particular of the age of 65 or more. This definition takes into account the fact that the average age varies between different populations, on different continents, etc. Most developed world countries have accepted the chronological age of 65 years as a definition of 'elderly' or older person (associated with the age at which one may begin to receive pension benefits), but like many westernized concepts, this does not adapt well to e.g. the situation in Africa. At the moment, there is no United Nations (UN) standard numerical criterion, but the UN agreed cut-off is 60+ years to refer to the older population in Western world. The more traditional African definitions of an elder or 'elderly' person correlate with the chronological ages of 50 to 65 years, depending on the setting, the region and the country.

In a further preferred embodiment said human preferably suffers from or is at (increased) likelihood of developing muscle decline such as sarcopenia or frailty. Frailty is a condition referring to a collection of symptoms or markers primarily due to the aging-related loss and dysfunction of skeletal muscle, such as: reduced physical activity, muscle weakness, decreased performance, physical weakness, poor endurance, exhaustion, slow walking speed, low muscle strength. In elderly, frailty will increase the risk of adverse events such as death, disability, and institutionalization. Disability refers to the inability to perform a physical activity. In one embodiment, the subject suffers from sarcopenia, loss of muscle mass related to aging. Sarcopenia is a muscle disease that may be acute or chronic and is defined as low muscle strength and low muscle mass (Cruz-Jentoft et al. "Sarcopenia: revised European consensus on definition and diagnosis" Age Ageing (2019) vol. 48(1) pages 16-31.

The nutritional composition according to the invention can advantageously be used for prevention and/or treatment of muscle decline, particularly for improving MPS. Muscle decline involves insufficient MPS. In the targeted population where muscle mass, strength and function is compromised, promoting MPS is a therapeutic use.

Preferably, the composition of the invention is for therapeutic use in improving whole body protein synthesis, preferably for use in improving MPS.

The invention may also be worded in terms of the use of a protein composition in the manufacture of a product for prevention and/or treatment of whole body protein metabolism decline or whole body protein function decline, or for use in improving whole body protein metabolism or whole body protein function, in a human subject, wherein the human subject is preferably an ageing human elderly subject of at least 40 years of age, wherein the composition comprises a protein fraction comprising 32-58wt% pea protein, 30-56wt% soy protein and 8-16wt% free leucine, preferably a protein fraction comprising 36-54wt% pea protein, 34-52wt% soy protein and 10 - 14wt% free leucine, and optionally up to 5wt% of free branched chain amino acids other than leucine, based on all proteinaceous matter, wherein the composition is essentially free from animal protein. Worded differently, the invention also relates to a method for prevention and/or treatment of whole body protein metabolism decline or whole body protein function decline, or for use in improving whole body protein metabolism or whole body protein function, in a human subject, wherein the human subject is preferably a an ageing human elderly subject of at least 40 years of age, wherein the subject is administered with a composition comprising a protein fraction comprising 32-58wt% pea protein, 30-56wt% soy protein and 8-16wt% free leucine, preferably a protein fraction comprising 36-54 wt% pea protein, 34-52wt% soy protein and 10 - 14wt% free leucine, and optionally up to 5wt% of free branched chain amino acids other than leucine, based on all proteinaceous matter, wherein the composition is essentially free from animal protein. The composition is detailed here below.

Within the above embodiments, the inventions is particularly directed to prevention and/or treatment of muscle decline, particularly for use in improving muscle mass, strength and function.

### Composition

The composition is plant-based and essentially free from animal protein, particularly essentially free from whey protein and casein (including caseinates and micellar casein). Particularly, the amount of animal protein is less than 1% of the protein fraction, most preferably there is no animal protein present in the composition. The composition comprises a protein fraction comprising 32-58wt% pea protein, 30-56wt% soy protein and 8-16wt% free leucine, preferably a protein fraction comprising 36-54wt% pea protein, 34-52wt% soy protein and 10 - 14wt% free leucine, and optionally up to 5wt% of free branched chain amino acids other than leucine, based on all proteinaceous matter. These numbers are based on a 30wt%, preferably a 20wt% variation on the protein fraction that was tested in the example, and showing MPS levels which are similar to the whey protein standard.

In a preferred embodiment, the composition comprises a protein fraction comprising 41-50wt% pea protein, 39-47wt% soy protein and 10.5 - 13.5wt% free leucine, based on all proteinaceous matter. In a more preferred embodiment, the composition comprises a protein fraction comprising 43-47wt% pea protein, 41-45wt% soy protein and 11 - 13wt% free leucine, based on all proteinaceous matter. These numbers are based on a 10% and 5% variation, respectively, based on the protein fraction tested in the example.

In a preferred embodiment, the sum of said proteins, free leucine and optionally free branched amino acids other than leucine equals 100 weight% of the protein fraction. In a more preferred embodiment, the protein fraction essentially consists of pea protein, soy protein and free leucine. In a preferred embodiment, the composition has no free amino acids, peptides or hydrolysates in the composition, except for free leucine.

The composition preferably induces MPS to an extent comparable (i.e. less than 10wt%, more preferably less than 5wt% deviation) to that of a composition having the same amount of protein but the protein fraction consisting of whey protein.

According to one embodiment, the nutritional composition according to the invention preferably contains between 10 and 30 gram of proteins per serving, more preferably between 15 and 25 grams serving. The serving is preferably in liquid form, preferably having a volume between 50 and 200 ml, preferably about 125 ml.

In the context of this application, when referring to a "protein mixture", a "protein fraction", "proteinaceous matter" or a "protein composition" according to the invention, is meant a collection of proteins, proteinaceous matter, peptides and amino acids, free or in any bound form. Hence, the protein fraction of a nutritional composition is the sum of all proteins, proteinaceous matter, peptides and amino acids, free or in any bound form present in the nutritional composition. Furthermore, the wording "protein mixture" refers to a collection of proteins, proteinaceous matter, peptides and amino acids as such, in any form, as well as to a collection of proteins, proteinaceous matter, peptides and amino acids simultaneously present in a matrix, such as an aqueous matrix, such as a liquid nutritional composition. In the latter case, the protein mixture may be referred to as a protein fraction of that matrix.

The proteins including soy protein and pea protein may be substantially in intact form or non-hydrolysed. However, the soy and/or pea protein may also be provided in the form of hydrolysates.

According to a preferred embodiment, the proteins including soy protein and pea protein are substantially in intact form or non-hydrolysed. In the context of the invention, a "non-hydrolysed" protein is equivalent to an "intact" protein, meaning that the protein has not been subjected to an hydrolysis process. However, minor amounts of hydrolysed proteins may be present in the source of non-hydrolysed proteins, or may be added to the formulation, such as additional amino acids. In a preferred embodiment, the only free amino acids added are branched amino acids leucine, isoleucine and valine.

In one embodiment of the present invention, the composition may, in addition to the free leucine, comprise another free amino acid, or a mixture of free amino acids other than leucine, up to about 1 g/100 ml, preferably up to about 0.5 gram/100 ml. The protein fraction preferably essentially consists of plant-based proteinaceous matter, in particular proteins, and free leucine. Small amounts of free amino acids other than free leu may be accounted for, preferably less than about 5%, more preferably less than about 2%, most preferably less than about 1% of the protein fraction. In a particularly preferred embodiment, there are no free amino acids (other than leucine), peptides or hydrolysates in the composition.

In the context of the invention, the term "about" should preferably be interpreted as a deviation of plus or minus 10 % of the given value.

According to a further embodiment, the nutritional composition according to the invention optionally comprises one or more of a fat fraction, a carbohydrate fraction, a dietary fibre fraction, and micronutrients.

In one embodiment of the present invention, the composition is in liquid form. The viscosity of the liquid nutritional composition is preferably lower than 500 mPa.s, measured at 20 °C (i.e. room temperature) at a shear rate of 100 s⁻¹, preferably between 10 and 200 mPa.s, more preferably between 10 and 100 mPa.s, most preferably below 50 mPa.s. The viscosity may suitably be determined using a rotational viscosity meter using a cone/plate geometry. This viscosity is ideal for enteral administration in the form of tube feeding or oral administration in the form of a drink or oral nutritional supplement.

In one embodiment of the present invention, the density of a liquid composition according to the invention ranges between 1.00 g/ml and 1.20 g/ml, especially between 1.05 g/ml and 1.15 g/ml. Most preferably, the liquid nutritional composition is sterilized or pasteurized. The liquid nutritional composition is provided in a ready to use liquid form and does not require reconstitution or mixing prior to use.

The composition of the invention may be adapted for tube feeding. The nutritional composition according to the invention preferably has the form of a complete food, i.e. it can meet all nutritional needs of the user. As such, the nutritional composition according to the invention preferably contains 1000 to 2500 kcal per daily dosage. Depending on the condition of the patient, a daily dose is about 25 to 35 kcal/kg bodyweight/day. Therefore, a typical daily dose for a 70 kg person contains about 2000 kcal.

The complete food can be in the form of multiple dosage units, e.g. from 8 (250 ml/unit) to 2 units (1 I/unit) per day for an energy supply of 2000 kcal/day using a liquid nutritional composition according to the invention of 1.0 kcal/ml.

Preferably, the nutritional composition is packaged, stored and provided in a tube feeding bag. Tube feeding is given to provide nutrition to patients which cannot obtain nutrition by swallowing, using a device such as a nasogastric feeding tube or a naso jejunal feeding tube, or by using a percutaneous endoscopic gastrostomy (PEG) or PEG-jejuno-feeding system. In the context of this application, the state of being fed by a feeding tube is called enteral feeding, comprising all of the abovementioned tube feeding systems, and the nutrition used in the feeding a feeding tube is called enteral nutrition. The (tube feed) composition preferably provides 1.0 - 1.8 g protein/kg body weight per day.

### Pea Protein

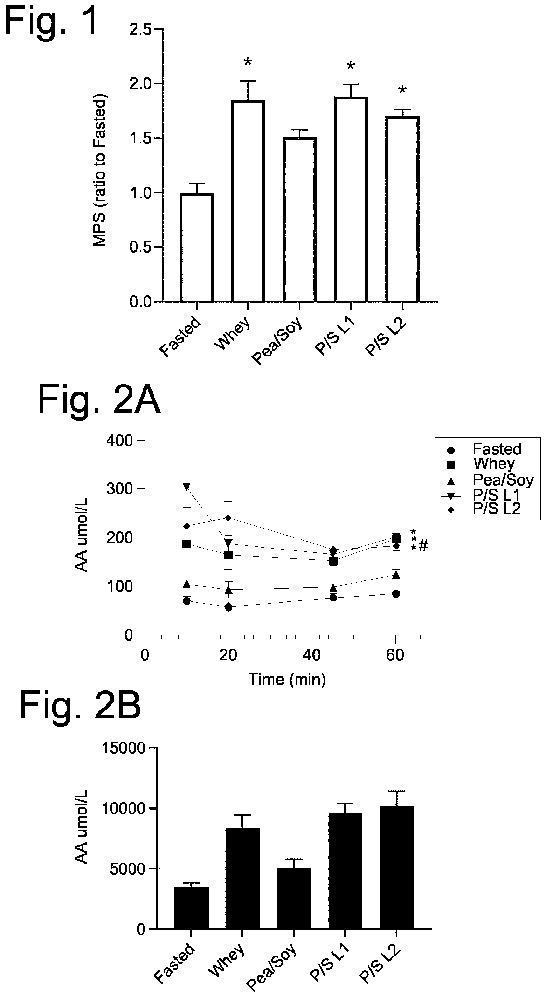

Compared to soy protein, pea protein advantageously reduces the amount of ANF in the plant-based composition. While pea and soy protein are similarly digestible (while both are inferior to whey protein), pea protein provides better digestibility of the amino acids lysine, phenylalanine and aspartic acid. Also, compared to soy protein, pea protein is low in terms of trypsin inhibitor activity. Reference is for instance made to Reinkensmeier et al. "Characterization of individual proteins in pea protein isolates and air classified samples", Food Research International 76 (2015) 160-167, and particularly figure 1 therein; trypsin inhibitor activity, expressed in trypsin inhibition units (activity per gram protein) is at least 5x higher for soy protein than it is for pea protein, even when allowing for some variation among pea and soy sources. By blending soy and pea protein in about similar amounts as proposed herein, a reduced ANF and particularly a reduced trypsin inhibiting effect (preferably at least 30% reduction in trypsin inhibition activity (TIA), compared to soy protein alone) is obtained, thus yielding enhanced digestibility of the plant-based mixture. TIA can be assessed for instance as addressed in Reikensmeijer (2015), its contents herewith incorporated by reference.

In the past, pea protein alone is generally classed as quite a poor plant-based source of protein, having a Biological Value (BV) of about 49 % when compared to e.g. whole egg (100 %), cow's milk (91 %), casein (77 %), soy (74 %) and wheat (54 %) (see e.g. Renner, E. (1983) Milk and dairy products in human nutrition. Volkswirtschaftlicher Verlag, Munich, Germany) and having a Protein Digestibility Corrected Amino acid Score (PDCAAS) which is 0.67, i.e. below the PDCAA for whole egg (1), cow's milk (1), casein (1) and soy (0.91). The BV of a protein is the amount of nitrogen used for tissue formation divided by the amount absorbed from the food and is expressed as a percentage. The AAS is the ratio between the amount of the first limiting amino acid in the protein under study (mg/g) and the amount of that amino acid in a reference protein (mg/g), optionally multiplied by the true digestibility (Protein Digestibility Corrected-AAS, PDCAA). According to the WHO (2007) recommendations on protein quality as the reference, pea has an amino acid score of below 1.0 due to the relatively low methionine content. Further, pea protein tastes quite bad (even in intact form) and it does not mix too well, leaving a kind of grainy texture to the protein.

Yet, in spite of the above, it was found that pea protein contributes to forming a good overall mix of amino acids. While whey proteins enter the blood stream very fast, pea proteins are absorbed much slower. Pea protein is quite high in cysteine content and can therefore compensate the inadequate amount of cysteine in casein proteins. Furthermore, pea protein is quite high in arginine compared to casein, soy or whey protein which is required for muscle metabolism and which facilitates the intake of body mass while reducing body fat; and it is quite high in lysine, which is needed to build protein muscle and assist in the maintenance of lean body mass.

Several pea sources are readily available to the skilled person, for example, from Roquette (Lestrem, France) which markets a pea isolate obtained from the yellow pea *(Pisum sativum),* and from Cosucra Groupe Warcoing (Warcoing, Belgium).

### Soy protein

Soy protein is categorized as a high-quality, complete protein although the methionine level is slightly below the WHO 2007 recommendation for methionine content. Soy proteins can be divided into different categories according to their production method. Soy protein isolate (SPI) is the most refined form of soy protein. Soy protein isolate contains about 90 percent protein. Soy protein concentrate (SPC) is basically soybean without the water soluble carbohydrates. It contains about 70 percent of protein. Several soy sources are readily available to the skilled person, for example, from The Solae Company (St. Louis, MO, USA).

It is preferred to use pea and soy protein sources with reduced amounts of anti-nutrition factors (ANFs) such as phytic acid, protease inhibitors, hemagglutinins, glucosinolates, tannins, and gossypol. These ANFs can interact with the plant-based proteins and negatively affect the digestibility of plant-based protein sources.

### Leucine

Although blending pea protein to soy protein results in reduced trypsin inhibiting properties and improved amino acid bioavailability, as evidenced in the experimental parts here below it is only with the addition of free leucine that MPS is induced to a satisfactory level, i.e. to the standard achieved with whey protein. In the calculations below it is demonstrated that the leucine derived from the plant-based blend has a bioavailability factor of 4.1, whereas the leucine from whey has a bioavailability factor of 15.4 and free leucine 17.4. Thus the leucine from whey protein results in a 3.8 x greater rise in blood leucine concentration relative to the plant-based blend, and the free leucine is 4.1 x more bioavailable. Adding the free leucine to the plant based blend brings this bioavailability factor of this blend up to the same level as whey.

It is preferred to maintain the total leucine levels provided by the composition (i.e. as free leucine or provided by the proteins and protein hydrolysates) in the range of 15.2 - 20.2 wt%, more preferably 17.1 - 20.9 wt%, most preferably 18.0 - 20.0 wt% total leucine, based on the weight of the protein fraction. L-leucine (in the application also called leucine, since the R-form of leucine is biologically not relevant in the context of this invention) is an essential amino acid, being part of a diverse number of proteins and, together with valine and isoleucine, belongs to the group of branched-chain amino acids.

The composition may comprises up to 5wt%, more preferably less than 2wt%, even more preferably less than 1wt% of the sum of free branched chain amino acids (BCAAs) other than leucine, i.e. valine and/or isoleucine, based on the weight of the protein fraction. It is prefered that the composition has a leucine:isoleucine: valine ratio of 2:0.8-1.2:0.8-1.2, in order to minimize transport competition between the BCAAs.

The composition may further comprise vitamin D. Vitamin D is a group of fat-soluble secosteroids, the two major physiologically relevant forms of which are vitamin D2 (ergocalciferol) and vitamin D3 (cholecalciferol). These are known collectively as calciferol. Vitamin D without a subscript refers to all forms of vitamin D, either D1, D2, D3 or D4, in particular D2 or D3, or any mixture thereof. According to one embodiment, vitamin D is used in an amount of 300 - 4,000 IU per daily dosage or serving.

### Exercise program

In a preferred embodiment, the human subject receiving the composition of the invention participates in a physical exercise program. The physical exercise regimen or program involves any physical exercise or activity other than or in addition to daily living activities that contributes to a negative energy balance or an activity that costs calories during that activity. Physical activity examples can be, but not limited to, resistance exercise, aerobic exercise or flexibility training or combinations thereof. Endurance training is not recommended, at least not as the sole source of physical exercise, for preserving muscle mass and ameliorating the progression of sarcopenia. Therefore, in one embodiment, the physical exercise regimen does not involve endurance training. It is preferred that the physical exercise regimen involves at least resistance exercise, attributed to its effects on muscle mass maintenance and stimulation of muscle mass increase. Activity patterns could vary from engaging in the above one or more physical activities for a minimum of 1 time per week but preferably 2 times per week and more preferably 3 or more times per week, including at least some form of resistance exercise. There are ACSM/AHA Guidelines for flexibility, endurance and resistance exercise which can be of help to the skilled person to determine a suitable physical exercise regimen, and these are considered incorporated by reference here. The physical exercise regimen involves daily physical exercise activities as described above, preferably comprising resistance exercise. The physical exercise regimen is preferably in accordance with ACSM/AHA guidelines, their contents herein incorporated by reference.

The invention will now be further elucidated by the examples here below, without being limited thereby.

### Example 1- MPS

MPS was assessed in ageing mice for possible intervention with:
(Group 2) 70 mg whey protein,
(Group 3) 70 mg of the sum of pea protein (P) and soy protein (S) [weight ratio 51:49],
(Group 4) 70 mg of the sum of P and S and free leucine (L1), with 31.5 mg pea protein, 30.1 mg soy protein, 8.4 mg free leucine [weight ratio P:S = 51:49, weight ratio P:S:L1 = 45:43:12],
(Group 5) 77.7 mg protein, i.e. 70 mg of the sum of P and S [51:49], 35.7 mg P, 34.2 mg S, and 7.7 mg free leucine (L2), weight ratio P:S:L2 = 46:44:10.
and compared to a fasted control (Group 1).

The amount of leu in Group 2 = 7.0 mg, and in Group 3 = 5.5 mg.

The total amount of leucine in L1 and L2 was similar, i.e. 19% of all proteinaceous matter (=13.22 mg).

The mice were orally forced fed (gavaged) with about 70 mg of total proteins, acutely and in 1 bolus. This is comparable with a human (person of 75 kg) equivalent dosing of 13 grams of protein. This amount of protein delivered to the mice has previously been shown as sufficient to trigger optimal MPS (van Dijk (2017) and Dijk (2018)).

It is generally accepted that whey protein is the standard in MPS.

### Animals

Aged male C57/BL6J mice of 25 months of age were obtained from Janvier Labs (Saint Berthevin, France). Animals were individually housed in a climate-controlled room (12:12 dark/light cycle (lights on: 7 a.m.) with a constant room temperature of 21±1°C). Housing consisted of Makrolon Type III cages (Techniplast, Italy) with bedding and tissues. Mice were fed ad libitum with a standard diet (AIN93M) and had free access to tap water. All experimental procedures were approved by an Animal Ethical Committee (DEC consult, Soest, the Netherlands) and complied with the principles of good laboratory animal care following the European Directive for the protection of animal used for scientific purposes. The animals were cared for according to the NIH Guide for the Care and Use of Laboratory Animals. Upon arrival, the mice were allowed to acclimatize for 2 weeks and were fasted 5 hours before section. At the day of section, mice were randomized to 5 groups (fasted, whey, Pea/Soy, P/S L1 and P/S L2) with no greater deviation than 5% from the overall mean bodyweight (fasted n=5, experimental groups n=15 per group).

### In vivo muscle protein synthesis (MPS)

MPS was measured with the SUnSET method as previously described by Goodman (2011), Goodman (2013), van Dijk (2017) and Dijk (2018). The SUnSET method is an alternative method to determine MPS compared to radioactive isotope or stable isotope tracers. Goodman et al. validated the SUnSET method with a 3H-phenylalanine flooding method in ex vivo plantaris muscle from mice that had received synergist ablation (SA). The results showed that the SUnSET technique was indistinguishable from a standard radioactive based or a standard stable isotope incorporation technique in detecting SA-induced increases in protein synthesis. In addition, in Salles (2013), protein synthesis was measured in myotubes after a 50 min incubation with L[1-¹³C]valine by measuring tracer enrichment, or a 30 min incubation with puromycin by quantifying incorporation of puromycin into peptides. Results of both methods showed comparable results on protein synthesis. Furthermore, the SUnSET technique was successfully applied in a previous study (Dijk(2018)) where similar dosages of protein showed an anabolic response in adult mice. This proves the reliability of the SUnSET method for measuring MPS.

At section day, mice received an oral gavage (end volume 0.5 mL) containing either total protein 69.90 mg whey (Lacprodan-9114) or 69.90 mg Pea/Soy (a mixture of 35.50 mg Pea (51%) and 34.10 mg Soy (49%)) or Pea/Soy (P/S) was enriched with 8.38 mg free Leucine = L1, resulting in less Pea (34.10 mg) and Soy (30.00 mg) or group 5: the additional Leucine (=7.72 mg) was added on top of the 69.90 mg total protein amount given, resulting in providing ^{~}10% more protein = L2. The postprandial conditions are compared with the fasting state (0.5 mL tap water) to indicate the levels of postprandial increase. 30 min after oral gavage adult mice received a s.c. injection with 0.04 mmol/g bodyweight puromycin (Calbiochem). After an additional 30 min, mice were euthanized by cardiac puncture under total isoflurane anaesthesia (isoflurane/N₂O/O₂). As a result, MPS was measured after a 60 min postprandial period. Plasma and serum samples were prepared by centrifugation and hind limb muscles were excised, weighted, frozen in liquid nitrogen and stored at -80°C until analysis.

### WES analysis

Left tibialis anterior muscles were cut into pieces and WB buffer (40 mM Tris pH 7.5; 1 mM EDTA; 5 mM EGTA; 10% glycerol; PhosSTOP Phosphatase Inhibitor Cocktail (Roche Diagnostics) and Protease Inhibitor Ultra Tablets (Roche Diagnostics), 1 tablet per 10 mL buffer) was added in a ratio of 15 µl/mg muscle. Muscles were homogenized using a FastPrep-24 (MP Biomedicals) with tubes containing ceramic beads and run 3 times for 30 s on 6.5 m/s speed. Between rounds, tubes were placed on ice to prevent heating of the samples. Subsequently, samples were continuously shaken at 4°C for 1 h and followed by another run with the FastPrep-24. Protein content was determined using the BCA protein assay kit (Pierce). Muscle homogenates were diluted in 0.1x sample buffer and diluted 0.2x with Fluorescent Master Mix (WES, ProteinSimple)), mixed and heated for 5 min at 100 °C according to suppliers instructions. 4 µg protein were loaded on the WES plate (ProteinSimple). For each experiment, a pool of the homogenates of mice in fasting state was made and loaded as a separate sample in each run to be able to compare samples from different runs. Primary anti-puromycin antibody 3RH11 (Kerafast), 5x diluted, anti-mouse secondary antibody (ProteinSimple) and luminol-peroxide solution were loaded to the WES plate. Protein separation, antibody staining and detection were performed with the WES using the 12-230 kDa WES Separation Module. In a separate run, the samples were run using a Total Protein protocol (ProteinSimple) to be able to correct for loading differences. Protein synthesis was determined by the luminescence of the whole lane using the Compass for SW software (ProteinSimple), corrected for total protein and calculated as a relative ratio to the fasted control group.

### Statistical analyses

All data are expressed as means±SEM. Statistical analyses were performed using IBM SPSS Statistics (version 19; SPSS Inc, Chicago, IL). Univariate ANOVA followed by SIDAK post hoc analysis was used to compare groups. Statistical significance is defined as P < 0.05.

### Results and discussion

After a 5h fast, the aged mice were supplemented with 1 bolus of a protein(blend). As the results in figure 1 show, mice supplemented with whey protein (group 2) had a significant increased MPS response, where Pea+Soy protein only (Group 3) had not. However, adding leucine to Pea+Soy protein was beneficial to overcome the anabolic threshold in aged mice (both Groups 4 and 5). During ageing the sensitivity of a postprandial response, especially towards protein is decreasing; hence the aged population requires more protein. In our model, providing the amount of 70 mg is sufficient to overcome this anabolic threshold. No difference was observed in the strategy how leucine was added: included in the total protein amount or on top of the total protein amount (L1 (Group 4) vs L2 (Group 5)); suggesting adding more plant-based proteins is not the way as alternative to whey, but the additional of leucine is.

While there was no group with free leucine in the present model, earlier work has demonstrated that there is no effect of leucine alone anticipated. In Dijk(2018), the same experimental protocol was used. Aged mice were supplemented with either whey protein enriched with leucine or with an equivalent amount of free leucine. It was demonstrated that the free leucine supplemented mice did not show an anabolic response, where the enriched-leucine whey group did. From these data it was concluded that aged mice require the building blocks and the anabolic stimulus to induce a MPS response, where in young rodents, only leucine may be sufficient.

### References:

1. Goodman, C.A. and T.A. Hornberger, Measuring protein synthesis with SUnSET: a valid alternative to traditional techniques? Exerc Sport Sci Rev, 2013. 41(2): p. 107-15.
2. Goodman, C.A., et al., Novel insights into the regulation of skeletal muscle protein synthesis as revealed by a new nonradioactive in vivo technique. FASEB J, 2011. 25(3): p. 1028-39.
3. Dijk, et al, 2018, Differential effects of leucine and leucine-enriched whey protein on skeletal muscle protein in aged mice, Clinical Nutrition ESPEN, 24, 127-133.
4. van Dijk, et al, 2017, Sarcopenia in older mice is characterized by a decreased anabolic response to a protein meal, Archives of Gerontology & Geriatrics, 69: 134-143.
5. Salles et al, 2013, 1,25(OH)2-vitamin D3 enhances the stimulating effect of leucine and insulin on protein synthesis rate through Akt/PKB and mTOR mediated pathways in murine C2C12 skeletal myotubes, Mol Nutr Food Res, 00, 1-10.

### Example 2 - leucine bioavailability

Aged mice (animal model: see example 1) were fed with one of the 4 supplements at T0 and after 10 minutes a droplet of blood from the tail was collected and stamped on a card. From these dry blood spots, the amino acid levels of leucine were determined at t = 10, 20, 45 and 60 minutes. The results are plotted in Figure 2A.

As Figure 2A shows, fasted mice did not show any increased levels of leucine and the 3 supplements showed significant increase of leucine in time, where the results with addition of free leucine were most profound. In Figure 2B the area under the curve of Figure 2A has been plotted, demonstrating that the protein supplements yielded similar leucine plasma levels for whey protein as well as for the pea+soy protein blend which was enriched with leucine. L1 and L2 showed similar numbers on leucine bioavailability.

Since feces was not collected, it was not possible to precisely calculate the N in- and output. However, with the known leucine plasma levels and the precise formulation of the supplements a credible assumption could be made of the bioavailability of leucine. Summarized, the amount of leucine present in the intact protein was known from the analyses of the pure protein sources (data sheets available). The leucine provided with the supplements is outlined in example 1.

From Figure 2A, the amount of plasma leucine at 10 - 20 minutes was obtained. Since fasted mice also show a baseline level of plasma leucine, these values were then deducted from the obtained leucine plasma values (column D in table below). In conclusion, these leucine levels in column D represented the increase from baseline levels. It was then calculated the contribution of the increase from the intact Leu (column E) and from the added free leucine (column F).

**Table: calculations on leucine bioavailability**

| Supplement | A* | B* | C* | D* | E* | F* | G* | H* |
|---|---|---|---|---|---|---|---|---|
| Fasted | 0 | 0 | 0 | | | | | |
| Whey | 7 | 0 | 7 | 107.5 | 15.4 | | 15.4 | **1.00** |
| Pea+soy | 5.5 | 0 | 5.5 | 22.5 | 4.1 | | 4.1 | **0.27** |
| Pea+soy+Leu (L1)^ | 4.84 | 8.38 | 13.22 | 167.5 | 3.6 | 17.3 | 12.7 | **0.83** |

| | | | | | | | | |
|---|---|---|---|---|---|---|---|---|
| * Explanations columns: A = leucine in supplement (mg) B = free leucine (mg) C = total leucine(mg) D = plasma level leucine-average 10-20 min compared to fasted (µmol/ml) E = relative plasma leucine increase from intact source (mmol/L/mg) F = relative plasma leucine increase from free amino acid source (mmol/L/mg) [taking the relative increase from the P/S group (4.1) away from the leucine response, and attribute the remaining response to the free leucine] G = total relative plasma leucine increase (mmol/L/mg) H = bioavailability factor (relative to whey protein) ^ Calculations for leucine bioavailability for L1 and L2 are the same. | | | | | | | | |

The data show that the bioavailability factor of whey protein (Group 1) was 15.4 (i.e. a rise of 15.4 mmol/L of plasma leucine levels is seen per mg of leucine consumed), whereas the bioavailability factor of the pea/soy blend (Group 2) was only 4.1. This indicates that the leucine present in whey protein was nearly 4 x more bioavailable than the leucine from the pea+soy protein blend. The bioavailability of free leucine was higher than that of whey protein (17.3 mmol/L/mg leucine). By adding the specified amount of free leucine it was possible to bring the bioavailability factor of the pea+soy+leucine blend (L1) to the equivalent of whey protein. It is clearly demonstrated in column H that the addition of free leucine advantageously contributes to the bioavailability and compensates for the limited bioavailability of leucine provided by a pea and soy protein blend, and therefore supports MPS response in these aged mice.

The results for L2 were similar to those of L1; induction of MPS was shown to be slightly better for L1.

### Example 3 - Composition according to the invention

A liquid nutritional product for improving muscle muscle mass, strength and function in an elderly person, the product comprising, per serving of 200ml, about 21 g proteinaceous matter, with 45wt% pea protein, 43wt% soy protein and 12wt% free leucine, calculated on the weight of the protein fraction. The total amount of leucine is about 19wt% of all protein. The product has a caloric content of 1.5 kcal/ml, and comprises about 15 wt% carbohydrates, 5.2wt% fat and 1.4 wt% fibre (31 en% fat, 39 en% carbohydrates, 28 en% protein and 2 en% fibre), minerals and vitamins.

## Claims

1. A nutritional composition for use in prevention and/or treatment of whole body protein metabolism decline or whole body protein function decline, or for therapeutic use in improving whole body protein metabolism or whole body protein function, in a human subject, wherein the human subject is an ageing human elderly subject of at least 40 years of age, wherein the composition comprises a protein fraction comprising 32-58 wt% pea protein, 30-56wt% soy protein and 8-16wt% free leucine, preferably a protein fraction comprising 36-54 wt% pea protein, 34-52 wt% soy protein and 10 - 14 wt% free leucine, and optionally up to 5 wt% of free branched chain amino acids other than leucine, based on all proteinaceous matter, wherein the composition is essentially free from animal protein.

2. The nutritional composition for use according to claim 1, wherein the composition comprises a protein fraction comprising 41-50wt% pea protein, 39-47wt% soy protein and 10.5 - 13.5wt% free leucine, based on all proteinaceous matter; wherein the composition preferably comprises a protein fraction comprising 43-47wt% pea protein, 41-45wt% soy protein and 11 - 13wt% free leucine, based on all proteinaceous matter.

3. The nutritional composition for use according to any one of the preceding claims, wherein the sum of said proteins, free leucine and optionally free branched amino acids other than leucine equals 100 weight% of the protein fraction.

4. The nutritional composition for use according to any one of the preceding claims, wherein the protein fraction essentially consists of pea protein, soy protein and free leucine.

5. The nutritional composition for use according to any one of the preceding claims, for use in prevention and/or treatment of muscle decline, or for use in improving muscle mass, strength and function.

6. The nutritional composition for use according to any one of the preceding claims, wherein the proteins are in hydrolysed or intact form, preferably in intact form.

7. The nutritional composition for use according to any of the preceding claims, wherein there are no free amino acids, peptides or hydrolysates in the composition, except for free leucine.

8. The nutritional composition for use according to any one of the preceding claims, wherein the human subject is a human elderly subject of at least 50 years of age, preferably at least 55 years of age; and/or wherein the human subject is an elderly human subject who is suffering or at (increased) risk of developing sarcopenia or frailty.

9. The nutritional composition for use according to any one of claims 4 - 8, wherein improving muscle mass, strength and function involves improving muscle protein synthesis (MPS).

10. The nutritional composition for use according to any one of the preceding claims, wherein the daily amount of protein administered to the human subject is between 1.0 and 1.8 g/kg body weight.

11. The nutritional composition for use according to any one of the preceding claims, wherein the composition is in liquid form, preferably adapted for tube feeding.

12. The nutritional composition for use according to any one of the preceding claims, wherein the amount of protein administered to the human subject is between 10 and 30 g per serving, preferably 15 - 25 g per serving.

13. The nutritional composition for use according to any one of the preceding claims, wherein the composition further comprises vitamin D.

14. A non-therapeutic method for improving muscle mass, strength and function in a healthy human subject, wherein the human subject is an ageing human elderly subject of at least 40 years of age, wherein the subject is administered with a composition comprising a protein fraction comprising 32-58wt% pea protein, 30-56wt% soy protein and 8-16wt% free leucine, preferably a protein fraction comprising 36-54wt% pea protein, 34-52wt% soy protein and 10 - 14wt% free leucine, and optionally up to 5wt% of free branched chain amino acids other than leucine, based on all proteinaceous matter, wherein the composition is essentially free from animal protein.

15. A nutritional composition, wherein the composition is essentially free from animal protein and comprises a protein fraction comprising 32-58 wt% pea protein, 30-56wt% soy protein and 8-16wt% free leucine, preferably a protein fraction comprising 36-54 wt% pea protein, 34-52 wt% soy protein and 10 - 14 wt% free leucine, and optionally up to 5 wt% of free branched chain amino acids other than leucine, based on all proteinaceous matter.

16. Nutritional composition according to claim 15, wherein the sum of said proteins, free leucine and optionally free branched amino acids other than leucine equals 100 weight% of the protein fraction.

17. Nutritional composition according to any of claim 15 or 16, wherein the weight ratio pea protein:soy protein:free leucine is 45:43:12 or 46:44:10.

## Patentansprüche

1. Nahrungszusammensetzung zur Anwendung bei der Vorbeugung und/oder Behandlung einer Abnahme des Ganzkörper-Proteinstoffwechsels oder einer Abnahme der Ganzkörper-Proteinfunktion oder zur therapeutischen Anwendung bei der Verbesserung des Ganzkörper-Proteinstoffwechsels oder der Ganzkörper-Proteinfunktion bei einem menschlichen Subjekt, wobei das menschliche Subjekt ein alterndes menschliches älteres Subjekt im Alter von mindestens 40 Jahren ist, wobei die Zusammensetzung eine Proteinfraktion umfasst, die 32-58 Gew.-% Erbsenprotein, 30-56 Gew.-% Sojaprotein und 8-16 Gew.-% freies Leucin umfasst, vorzugsweise eine Proteinfraktion, die 36-54 Gew.-% Erbsenprotein, 34-52 Gew.-% Sojaprotein und 10-14 Gew.-% freies Leucin umfasst, und optional bis zu 5 Gew.-% an freien verzweigtkettigen anderen Aminosäuren als Leucin, bezogen auf die gesamte proteinhaltige Masse, wobei die Zusammensetzung im Wesentlichen frei von tierischem Protein ist.

2. Nahrungszusammensetzung zur Anwendung nach Anspruch 1, wobei die Zusammensetzung eine Proteinfraktion umfasst, die 41-50 Gew.-% Erbsenprotein, 39-47 Gew.-% Sojaprotein und 10,5-13,5 Gew.-% freies Leucin, bezogen auf die gesamte proteinhaltige Masse, umfasst; wobei die Zusammensetzung vorzugsweise eine Proteinfraktion umfasst, die 43-47 Gew.-% Erbsenprotein, 41-45 Gew.-% Sojaprotein und 11-13 Gew.-% freies Leucin, bezogen auf die gesamte proteinhaltige Masse, umfasst.

3. Nahrungszusammensetzung zur Anwendung nach einem der voranstehenden Ansprüche, wobei die Summe der genannten Proteine, des freien Leucins und optional der freien verzweigtkettigen anderen Aminosäuren als Leucin 100 Gew.-% der Proteinfraktion entspricht.

4. Nahrungszusammensetzung zur Anwendung nach einem der voranstehenden Ansprüche, wobei die Proteinfraktion im Wesentlichen aus Erbsenprotein, Sojaprotein und freiem Leucin besteht.

5. Nahrungszusammensetzung zur Anwendung nach einem der voranstehenden Ansprüche, zur Anwendung bei der Vorbeugung und/oder Behandlung von Muskelschwund oder zur Anwendung bei der Verbesserung von Muskelmasse, -kraft und -funktion.

6. Nahrungszusammensetzung zur Anwendung nach einem der voranstehenden Ansprüche, wobei die Proteine in hydrolysierter oder intakter Form vorliegen, vorzugsweise in intakter Form.

7. Nahrungszusammensetzung zur Anwendung nach einem der voranstehenden Ansprüche, wobei in der Zusammensetzung keine freien Aminosäuren, Peptide oder Hydrolysate enthalten sind, mit Ausnahme von freiem Leucin.

8. Nahrungszusammensetzung zur Anwendung nach einem der voranstehenden Ansprüche, wobei das menschliche Subjekt ein menschliches älteres Subjekt im Alter von mindestens 50 Jahren ist, vorzugsweise im Alter von mindestens 55 Jahren; und/oder wobei das menschliche Subjekt ein menschliches älteres Subjekt ist, das an Sarkopenie oder Gebrechlichkeit leidet oder ein (erhöhtes) Risiko aufweist, diese zu entwickeln.

9. Nahrungszusammensetzung zur Anwendung nach einem der Ansprüche 4 - 8, wobei die Verbesserung von Muskelmasse, -kraft und -funktion die Verbesserung der Muskelproteinsynthese (MPS) beinhaltet.

10. Nahrungszusammensetzung zur Anwendung nach einem der voranstehenden Ansprüche, wobei die dem menschlichen Subjekt verabreichte tägliche Proteinmenge zwischen 1,0 und 1,8 g/kg Körpergewicht liegt.

11. Nahrungszusammensetzung zur Anwendung nach einem der voranstehenden Ansprüche, wobei die Zusammensetzung in flüssiger Form vorliegt, vorzugsweise geeignet für eine Sondenernährung.

12. Nahrungszusammensetzung zur Anwendung nach einem der voranstehenden Ansprüche, wobei die dem menschlichen Subjekt verabreichte Proteinmenge zwischen 10 und 30 g pro Portion, vorzugsweise 15-25 g pro Portion, beträgt.

13. Nahrungszusammensetzung zur Anwendung nach einem der voranstehenden Ansprüche, wobei die Zusammensetzung weiter Vitamin D umfasst.

14. Nicht-therapeutisches Verfahren zur Verbesserung von Muskelmasse, -kraft und -funktion bei einem gesunden menschlichen Subjekt, wobei das menschliche Subjekt ein alterndes menschliches älteres Subjekt im Alter von mindestens 40 Jahren ist, wobei dem Subjekt eine Zusammensetzung verabreicht wird, die eine Proteinfraktion umfasst, die 32-58 Gew.-% Erbsenprotein, 30-56 Gew.-% Sojaprotein und 8-16 Gew.-% freies Leucin umfasst, vorzugsweise eine Proteinfraktion, die 36-54 Gew.-% Erbsenprotein, 34-52 Gew.-% Sojaprotein und 10-14 Gew.-% freies Leucin umfasst, und optional bis zu 5 Gew.-% an anderen freien verzweigtkettigen Aminosäuren als Leucin, bezogen auf die gesamte proteinhaltige Masse, wobei die Zusammensetzung im Wesentlichen frei von tierischem Protein ist.

15. Nahrungszusammensetzung, wobei die Zusammensetzung im Wesentlichen frei von tierischem Protein ist und eine Proteinfraktion umfasst, die 32-58 Gew.-% Erbsenprotein, 30-56 Gew.-% Sojaprotein und 8-16 Gew.-% freies Leucin umfasst, vorzugsweise eine Proteinfraktion, die 36-54 Gew.-% Erbsenprotein, 34-52 Gew.-% Sojaprotein und 10-14 Gew.-% freies Leucin umfasst, und optional bis zu 5 Gew.-% an anderen freien verzweigtkettigen Aminosäuren als Leucin, bezogen auf die gesamte proteinhaltige Masse.

16. Nahrungszusammensetzung nach Anspruch 15, wobei die Summe der genannten Proteine, des freien Leucins und optional der anderen freien verzweigtkettigen Aminosäuren als Leucin 100 Gew.-% der Proteinfraktion beträgt.

17. Nahrungszusammensetzung nach einem der Ansprüche 15 oder 16, wobei das Gewichtsverhältnis Erbsenprotein:Sojaprotein:freies Leucin 45:43:12 oder 46:44:10 beträgt.

## Revendications

1. Composition nutritionnelle destinée à être utilisée dans la prévention et/ou le traitement du déclin du métabolisme protéique du corps entier ou du déclin de la fonction protéique du corps entier, ou destinée à une utilisation thérapeutique dans l'amélioration du métabolisme protéique du corps entier ou de la fonction protéique du corps entier, chez un sujet humain, où le sujet humain est un sujet humain vieillissant âgé d'au moins 40 ans, où la composition comprend une fraction protéique comprenant de 32 à 58 % en poids de protéine de pois, de 30 à 56 % en poids de protéine de soja et de 8 à 16 % en poids de leucine libre, de préférence une fraction protéique comprenant de 36 à 54 % en poids de protéine de pois, de 34 à 52 % en poids de protéine de soja et de 10 à 14 % en poids de leucine libre, et facultativement jusqu'à 5 % en poids d'acides aminés à chaîne ramifiée libres autres que la leucine, par rapport à la matière protéique totale, où la composition est essentiellement exempte de protéine animale.

2. Composition nutritionnelle pour une utilisation selon la revendication 1, où la composition comprend une fraction protéique comprenant de 41 à 50 % en poids de protéine de pois, de 39 à 47 % en poids de protéine de soja et de 10,5 à 13,5 % en poids de leucine libre, par rapport à la matière protéique totale ; où la composition comprend de préférence une fraction protéique comprenant de 43 à 47 % en poids de protéine de pois, de 41 à 45 % en poids de protéine de soja et de 11 à 13 % en poids de leucine libre, par rapport à la matière protéique totale.

3. Composition nutritionnelle pour une utilisation selon l'une quelconque des revendications précédentes, où la somme desdites protéines, de la leucine libre et facultativement des acides aminés ramifiés libres autres que la leucine, est égale à 100 % en poids de la fraction protéique.

4. Composition nutritionnelle pour une utilisation selon l'une quelconque des revendications précédentes, où la fraction protéique est essentiellement composée de protéine de pois, de protéine de soja et de leucine libre.

5. Composition nutritionnelle pour une utilisation selon l'une quelconque des revendications précédentes, destinée à être utilisée dans la prévention et/ou le traitement du déclin musculaire, ou destinée à être utilisée dans l'amélioration de la masse, la force et la fonction musculaires.

6. Composition nutritionnelle pour une utilisation selon l'une quelconque des revendications précédentes, où les protéines sont sous forme hydrolysée ou intacte, de préférence sous forme intacte.

7. Composition nutritionnelle pour une utilisation selon l'une quelconque des revendications précédentes, où la composition ne comprend pas d'acides aminés libres, de peptides ou d'hydrolysats, à l'exception de la leucine libre.

8. Composition nutritionnelle pour une utilisation selon l'une quelconque des revendications précédentes, où le sujet humain est sujet humain âgé d'au moins 50 ans, de préférence au moins 55 ans ; et/ou où le sujet humain est un sujet humain âgé qui souffre ou présente un risque (accru) de développer une sarcopénie ou une fragilité.

9. Composition nutritionnelle pour une utilisation selon l'une quelconque des revendications 4 à 8, où l'amélioration de la masse, la force et la fonction musculaires implique l'amélioration de la synthèse des protéines musculaires (MPS).

10. Composition nutritionnelle pour une utilisation selon l'une quelconque des revendications précédentes, où la quantité quotidienne de protéines administrées au sujet humain est comprise entre 1,0 et 1,8 g/kg de poids corporel.

11. Composition nutritionnelle pour une utilisation selon l'une quelconque des revendications précédentes, où la composition est sous forme liquide, de préférence adaptée à l'alimentation par sonde.

12. Composition nutritionnelle pour une utilisation selon l'une quelconque des revendications précédentes, où la quantité de protéines administrées au sujet humain est comprise entre 10 et 30 g par portion, de préférence entre 15 et 25 g par portion.

13. Composition nutritionnelle pour une utilisation selon l'une quelconque des revendications précédentes, où la composition comprend en outre de la vitamine D.

14. Procédé non-thérapeutique pour l'amélioration de la masse, la force et la fonction musculaires chez un sujet humain sain, où le sujet humain est un sujet humain vieillissant âgé d'au moins 40 ans, où le sujet se voit administrer une composition comprenant une fraction protéique comprenant de 32 à 58 % en poids de protéine de pois, de 30 à 56 % en poids de protéine de soja et 8 à 16 % en poids de leucine libre, de préférence une fraction protéique comprenant de 36 à 54 % en poids de protéine de pois, de 34 à 52 % en poids de protéine de soja et 10 à 14 % en poids de leucine libre, et facultativement jusqu'à 5 % en poids d'acides aminés à chaîne ramifiée libres autres que la leucine, par rapport à la matière protéique totale, où la composition est essentiellement exempte de protéine animale.

15. Composition nutritionnelle, où la composition est essentiellement exempte de protéine animale et comprend une fraction protéique comprenant de 32 à 58 % en poids de protéine de pois, de 30 à 56 % en poids de protéine de soja et de 8 à 16 % en poids de leucine libre, de préférence une fraction protéique comprenant de 36 à 54 % en poids de protéine de pois, de 34 à 52 % en poids de protéine de soja et de 10 à 14 % en poids de leucine libre, et facultativement jusqu'à 5 % en poids d'acides aminés à chaîne ramifiée libres autres que la leucine, par rapport à la matière protéique totale.

16. Composition nutritionnelle selon la revendication 15, où la somme desdites protéines, de la leucine libre et facultativement des acides aminés ramifiés libres autres que la leucine est égale à 100 % en poids de la fraction protéique.

17. Composition nutritionnelle selon l'une quelconque des revendications 15 ou 16, où le rapport pondéral protéine de pois : protéine de soja : leucine libre est de 45 :43 :12 ou de 46 :44 :10.
